# EUROPEAN PATENT SPECIFICATION

(11) **EP 4 338 706 B1**
(45) Date of publication and mention of the grant of the patent: **29.04.2026**
(21) Application number: 22195652.7
(22) Date of filing: 14.09.2022
(51) Int. Cl.: A61F 2/16

(54) **INTRAOCULAR LENS**
INTRAOKULARLINSE
LENTILLE INTRAOCULAIRE

(43) Date of publication of application: 20.03.2024
(73) Proprietor: Soleko S.p.A., 03037 Pontecorvo (FR) (IT)
(72) Inventor: SCALORBI, Luca, 03043 Cassino (FR) (IT)
(74) Representative: Papa, Elisabetta

(56) References cited:
- US-A- 4 249 271
- US-A1- 2004 015 235
- ROSSI TOMMASO ET AL: "A novel intraocular lens designed for sutureless scleral fixation: surgical series", GRAEFE'S ARCHIVE FOR CLINICAL AND EXPERIMENTAL OPHTHALMOLOGY, vol. 259, no. 1, 1 January 2021 (2021-01-01) - 1 January 2021 (2021-01-01), pages 257 - 262, XP037331613, ISSN: 0721-832X, DOI: 10.1007/S00417-020-04789-3

## Description

The present invention relates to an intraocular lens, in particular for posterior chamber sutureless scleral fixation.

### Background

The human eye provides vision by refracting light through a clear outer portion (cornea) and by way of a crystalline lens onto a retina. The quality of the focused image depends on many factors including the size and shape of the eye, and the transparency of the cornea and the crystalline lens. When age or disease causes the crystalline lens to become clouded, vision deteriorates because of the loss of retinal image quality. This loss of optical quality in the lens of the eye is medically known as cataract. An accepted treatment for this condition is surgical removal of the lens and replacement of the crystalline lens function by an artificial intraocular lens (IOL).

The majority of cataractous lenses are removed by a surgical technique called phacoemulsification. During this procedure, a portion of the anterior capsule is removed and a thin phacoemulsification cutting tip is inserted into the diseased lens and vibrated ultrasonically. The vibrating cutting tip liquefies or emulsifies the nucleus and cortex of the crystalline lens so that it may be aspirated out of the eye. The diseased nucleus and cortex of the crystalline lens, once removed, is replaced by an artificial intraocular lens (IOL) in the remaining capsule (in-the-bag).

The implant of an intraocular lens (IOL) is the mainstay of cataract surgery and secondary aphakia correction, often achieving substantial visual rehabilitation. Whenever possible, elective in-the-bag IOL positioning offers unparalleled stability and optimal effective lens placement. In case of insufficient or non-existing capsular support as in some circumstances including trauma, complicated surgery, and other ocular comorbidities such as Marfan syndrome, pseudoexfoliation syndrome, and megalocornea, multiple alternative solutions have been deployed. IOL can be placed in the anterior chamber (AC) using an anterior chamber IOL (ACIOL), or in the posterior chamber (PC) with iris-fixated IOLs (IFIOLs) and scleral-fixated IOLs (SFIOLs).

ACIOL and iris claw IOL are easy and fast to implant but might be associated with persistent inflammation, cystoid macular edema, and progressive endothelial cell loss with corneal decompensation. Various techniques have been proposed for scleral fixation of IOL. Classically, a rigid polymethyl methacrylate IOL is fixated to the sclera with prolene sutures. However, this procedure requires a large corneal incision, long operating times, and might be associated with late IOL dislocation due to loosening of sutures or erosion. Furthermore, sutured scleral IOL is associated with significant optic tilt in over 50% of cases.

In the last few years, other techniques have been proposed for sutureless scleral fixation of a three-piece IOL with either fibrin glue or by tucking the haptics into scleral tunnels or pockets. Scleral gluing fixation technique has also been successfully combined with iris repair surgery.

Theoretically, PC-IOLs (posterior chamber IOLs) could represent the preferred choice because they have an optical advantage due to their more natural anatomic location and proximity to the nodal point. The original technique for PC-IOL implantation, described by Malbran et al during the 1980s, entailed scleral suturing, a long learning curve, the need for a large corneal incision, and exposure to the risk of postoperative complications related to suture degradation and breakage. This led to research with the aim of developing new solutions, including sutureless techniques. However, these required greater efforts from the surgeon, probably because of the lack of IOLs specially designed for this purpose.

PC IOLs have the advantage of minimize the risk of glaucoma and bullous keratopathy because the lens is located away from the anterior segment structures. Surgeons prefer posterior chamber IOLs because they cause less endothelial damage than anterior chamber IOLs, and there is a tendency to use foldable IOLs to reduce surgically induced astigmatism and improve refractive outcomes.

Since SFIOLs were introduced, the technique and materials have been modified to improve success rate and reduce the risk of complications. Sutureless scleral fixation presents several advantages, since it reduces suture-related risks, such as suture degradation, endophthalmitis, erosion, haemorrhage, chronic inflammation, IOL tilting, damaging or displacement. Scleral fixation of a posterior chamber IOL can be achieved through several techniques, such as scleral fixation approaches using non-absorbable sutures, sutureless scleral fixation by means of a trans-scleral tunnel, and fibrin glue, needle-guided, and flanged IOL implantation. Sutureless trans-scleral fixation was initially introduced by Maggi et al. in 1997, followed by the tunnel fixation method, proposed by Gabor Scharioth, and later modified as glued transscleral fixation by Agarwal et al. Recently, Yamane proposed "flanged fixation". This so-called Yamane technique externalizes the haptics of a three-piece IOL using a thin-walled 30- or 27-gauge needle inserted through two transconjunctival sclerotomies. Each haptic of the IOL is carefully placed into the lumen of the needle using intraocular forceps. Then, the needle is used to externalize each haptic on the conjunctival surface, followed by low temperature cautery to make a flange or bulb at the edge of the haptics. This flange prevents the haptics from prolapsing back into the posterior chamber. Thus, the IOL is fixated efficiently in the posterior segment in the absence of capsular support. Moreover, scleral-fixated IOL implantation is considered to be a safe procedure, in that the results show a reduction in suture-related complications and in induced astigmatism.

The goal of these techniques is to symmetrically fixate the two haptics of a three-piece IOL to the scleral tissue. However, the use of an IOL that is not specifically designed for sutureless intrascleral fixation could result in haptic torsion and optic plate tilting, causing ocular aberrations, iris chafing, and chronic inflammation.

US4249271A1 discloses an improved intraocular-lens structures for use as implants in ophthalmological surgery, the lens being a replacement for a cataract-clouded natural lens, and the replacement being installed in the pupil at the iris as the operative step following removal of the cataracted lens. The lens features adapter structure assembled to an optically finished lens element and including plural angularly spaced stabilizing feet which are formed integrally with the body of the adapter and which are axially offset from the adapter body to permit the stabilizing feet and the adapter body to engage opposite sides of the iris.

US20040015235A1 discloses an intraocular lens including an optical portion of a transparent, deformable material, at least one haptic radially projecting from the optical portion for supporting the optical portion in a position parallel to and against an anterior iris surface plane, and at least one aperture bounded by the haptic. At least a stiff portion of the haptic has a higher stiffness against bending about an axis in the radial direction than the optical portion. furthermore, the stiff portion has a width (a) measured parallel to the plane and perpendicular to the radial direction, which is smaller than the size (b) of the optical portion in the direction of the width.

ROSSI TOMMASO et al, "A novel intraocular lens designed for sutureless scleral fixation: surgical series", GRAEFE'S ARCHIV FOR CLINICAL AND EXPERIMENTAL OPHTHALMOLOGY, discloses an intraocular lens featuring T-shaped harpoons protruding off the closed haptics in order to allow self-anchoring on the sclera without the need for sutures.

Therefore, the object of the present invention is to solve the problems still left open by the known art and this is obtained through a specially designed sutureless trans-scleral fixation IOL, as defined in claim 1.

Additional features of the subject invention device are set forth in the corresponding depending claims.

The present invention, by overcoming the problems of known art, involves several and evident advantages.

### Brief description of the figures

These and other advantages, together with the features and use modes of the present invention, will result evident from the following detailed description of preferred embodiments thereof will result evident from the following detailed description of preferred embodiments thereof, shown by way of example and not with limitative purposes, by making reference to the figures of the enclosed drawings, wherein:
- Figure 1 is a perspective view of a lens according to a first embodiment of the present invention;
- Figure 2 is a plan view of the lens of Figure 1;
- Figure 3 is a sectional view of the lens of figure 1 taken along the line A-A of Figure 2;
- Figure 4 is a perspective view of the lens according to a second embodiment of the present invention;
- Figure 5 is a plan view of the lens of Figure 5;
- Figure 6 is a sectional view of the lens of Figure 5 taken along the line A-A of Figure 5;
- Figures 7, 8 show two possible additional embodiments of the invention;
   and
- Figure 9A to 9E show details of the surgical technique.

### Detailed description of the invention

The present invention will be described in details hereinafter by referring to the above-mentioned figures.

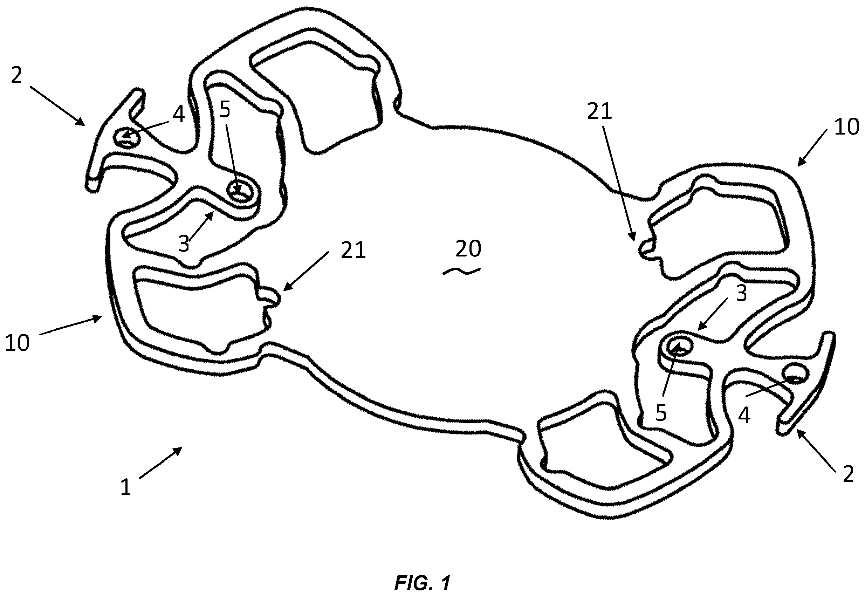

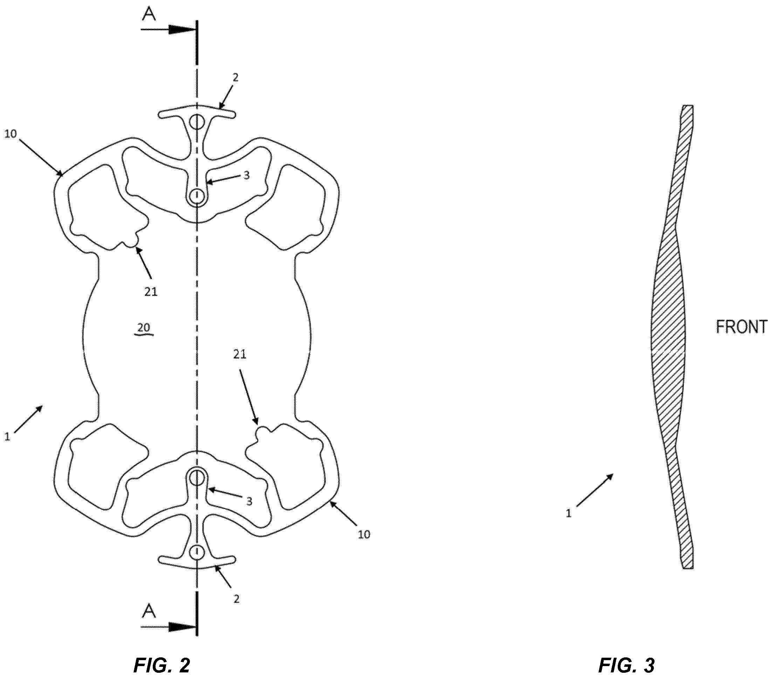

Figure 1 to 3 show a first embodiment of a lens according to the invention.

An IOL 1 according to the invention is a foldable IOL designed for posterior chamber sutureless scleral fixation. The lens has two haptics 10. At the extremity of the two haptics, flexible plugs 2 are positioned, which need to be anchored to the sclera. The plugs 2 prevent the backward movement of the haptic into the eye after the scleral fixation.

The lens according to this first embodiment of the invention is a one-piece IOL that can be made by a hydrophilic acrylic foldable material.

Preferably, it is characterized by an optic plate diameter between 5-8 mm and an overall diameter included in 11-15 mm.

The lens has two haptics 10. Preferably, the haptics 10 have an anterior angulation of 0-13° with respect to the optic plate. They have also two small incisions (markers) 21 that allow surgeons to quickly check proper side after unfolding of the lens. The distal part of the haptics is designed to create a four-points stabilization contact.

The lens design assures the anchoring to the scleral tissue by means of trans-scleral plugs 2 without the need of suture stitches, leading to a simpler operation and reduced surgery time.

According to this first embodiment, the lens 1 comprises two foldable T-shaped trans-scleral plugs 2, that allow anchorage to the sclera without suture. The IOL can be injected via a dedicated disposable plunger injector with a tip of 2.1-2.7 mm and cartridge through a 2.3-2.9 mm corneal incision.

The traditional surgical technique for IOL implantation requires the opening of the conjunctiva, the creation of two scleral flaps and two sclerotomies 180 degrees apart. The lens is injected into the anterior chamber through a corneal tunnel, and the two plugs are externalized through the sclerotomies under the scleral flaps using two crocodile tip forceps, with the so-called 'handshake technique'. The sutureless trans-scleral fixation technique used with a lens according to the invention has shown good postoperative outcomes, few intraoperative complications, and reduction in surgical complexity, as reported by several authors. The advantages of the surgical technique are the absence of haptic manipulation, self-centration, and firm fixation of the IOL.

The lens is usually implanted in the ciliary sulcus in cases of aphakia and cataract surgery, but, according to the literature, other clinical cases in which the lens implant is used are: IOL subluxation/dislocation, dropped nucleus, anisometropia, retinal detachment, traumatic cataract, Marfan's syndrome.

In order to reduce the rupture risk and minimize the tool contact with the optic plate, the lens 1 comprises in particular:
1. One hole 4 on each trans-scleral plug 2;
2. Two tabs 3, as extensions of the two plugs 2, each one having a hole 5;

Each trans-scleral plug 2 hosts one hole 4 on its T-neck which has to be intercepted with a hook to grab and move the IOL during the surgery, particularly after the expulsion of the device from the injector. Holes 4 are meant to avoid the crocodile clip or ILM forceps pressure on the T-shaped plugs 2, because there's the risk of generating a tensional stress on the T-neck leading to the plug weakening or rupture during the surgery. The second intercepted plug is usually grabbed by touching and moving the optic plate with the alligator clip/ILM forceps, risking the induction of a scratch on the optic surface. For this reason, two tabs 3 hosting holes 5 have been designed as a protrusion of each T-shaped plug 2 to the direction of the optic plate.

This component has been developed in order to grab the hole and consequently the second plug with a hook, in an easier way and in order to avoid the touching of the optic plate. These tabs 3 preserve the symmetrical design of the IOL, which remains versatile and adaptable to the requirements of each surgeon. Moreover, the presence of a tab also on the leading plug, can let it go outside the injector tip without tilting: being a protrusion of the plug, it works as a stabilizer, minimizing overturning events. The lens, being symmetrical, can be preloaded or manually loaded in the injection system, because its position in the cartridge does not affect the surgical procedure.

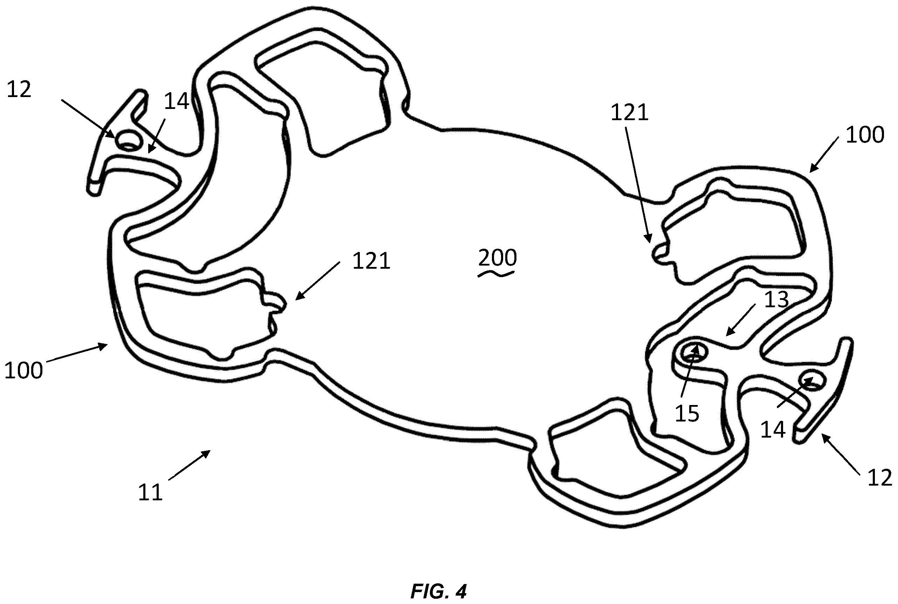

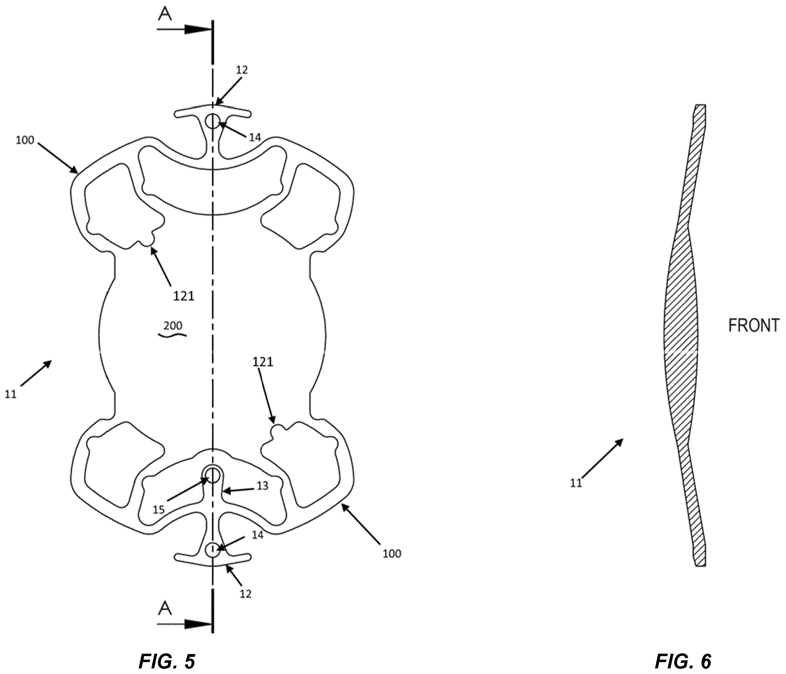

Figures 4 to 6 refer to a second embodiment of the present invention. In particular, with respect to the first embodiment, the second embodiment refers to a lens 11 having two plugs 12 with respective holes 14, but only one tab 13 with respective hole 15, so realizing an asymmetrical IOL design, with only one tab. In this case, the lens should be preloaded in the injector in order to simplify the procedure for the surgeon and the operating room staff: the asymmetrical design, in case of not-preloaded lens, could lead to misunderstandings at the moment of the loading of the lens.

According to the invention, the lens of both the embodiments can also be made of a hydrophobic material (instead of a hydrophilic one). Hydrophilic acrylic materials, according to scientific literature, have the following disadvantages:
- High rate of posterior capsule opacification
- High rate of anterior capsule opacification
- Greater lens epithelial cell ongrowth on the lens surface

In order to overcome these problems, the lens according to the invention can be created with a hydrophobic material, preserving the original design. The hydrophobic material makes the IOL more compatible with the use of silicone oil, gas and all the procedures where the device can encounter dryness.

According to the present invention, an intraocular lens may be realized so to have not transparent or coloured portions at the haptics, in order to be more visible during the surgical intervention.

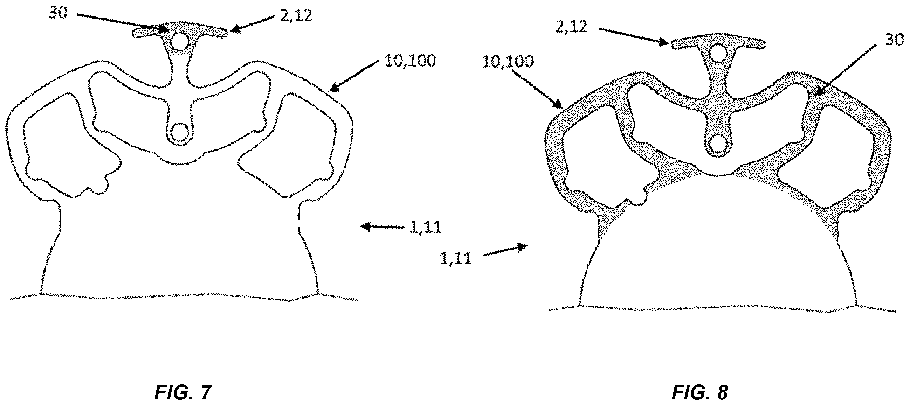

For example, one or both the haptics 10, 100 can be coloured or, preferably, only one or both the tabs 2, 12 can be coloured, as shown in figures 7, 8.

The colouring can be obtained by addition of a dye to the composition of the material used for making of the lens, or by bounding the tinted material to the clear one. The colouring can by total or can cover partially the thickness of the lens.

In another embodiment, the portions are made visible by applying a superficial texture such as coarse or satin finishings.

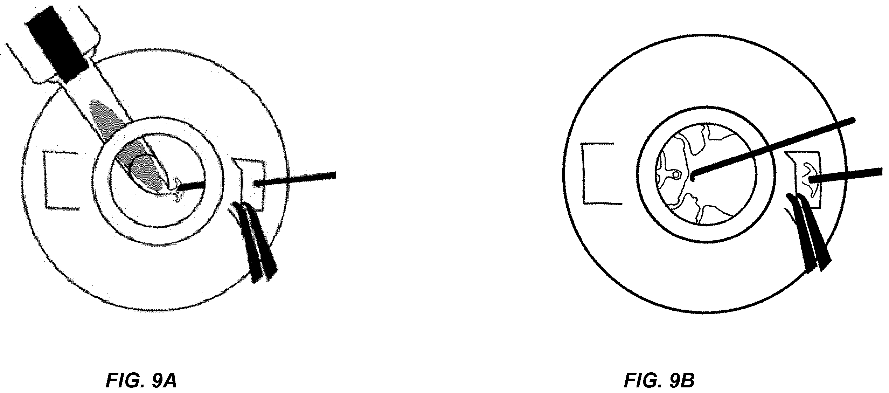

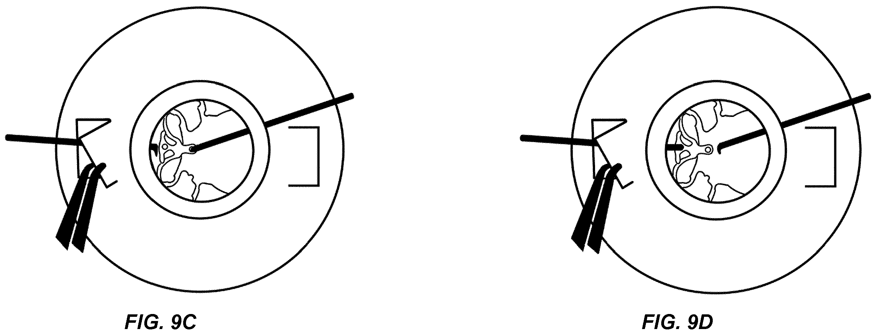

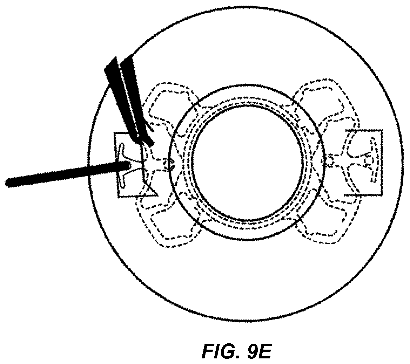

Surgical steps that have to be performed to obtain a good implantation of the presented intraocular lens is described here below and depicted in figures 9A to 9E.
1. Conjunctival dissection at 2 mm from limbus: it is less traumatic for conjunctiva and improves closure and recovery;
2. 3.5 mm x 3.5 mm scleral flaps dissection is recommended if sclera or conjunctiva are very thin and to avoid future plug extrusion; flaps incisions must be as deep as ½ or 2/3 of the sclera's thickness and lined up 180° from each other.
3. Complete pars plana vitrectomy and eventual IOL removal including IOL suction/grasping/cutting at vitreous cavity or limbus;
4. 1.5 mm from posterior surgical limbus sclerotomies that must be parallel to iris plane to reach the sulcus;
5. Enlarged 25G sclerotomies and IOL injection: it's important not to have forceps resistance at sclerotomies;
   a) When the leading plug comes out from the injector tip, it must be grabbed through the hole on the T-stem by a hook (Figure 9A);
   b) Check IOL position and turn it if needed, into the vitreous cavity;
   c) Anchor the leading plug to the sclera (Figure 9B);
   d) Once the IOL is completely outside the injector, the second plug is hidden behind the iris, so the tab's hole connected to the second plug must be intercepted with a hook in order to provide second plug visibility behind the pupil (Figure 9C);
   e) Once the second plug is visible, it must be grabbed through the hole on the T-stem by a second hook and anchored to the sclera (Figures 9D, 9E).
6. Suture corneal incision and scleral flaps;
7. Viscoelastic cleaning;
8. Inner haptics enclavation at sulcus: push the IOL anteriorly to engage inner haptics at sulcus;
9. Suture sclerotomies.

In case of using the asymmetrical design with one tab, the surgical procedure would be the same of the one descripted for the two-tabs design

The present invention has been so far described with reference to preferred embodiments thereof. It is to be meant that each one of the technical solutions implemented in the preferred embodiments, described herein by way of example, can advantageously be combined, differently from what described, with the other ones, to create additional embodiments, belonging to the same inventive core and however all within the protective scope of the here below reported claims.

## Claims

1. An intraocular lens (1, 11) for posterior chamber sutureless scleral fixation, comprising an optic plate (20, 200) and two haptics (10, 100), each haptic (10, 100) having, at its extremity, a respective plug (2, 12) for the anchoring to the sclera, each plug (2, 12) bearing a hole (4, 14);
**characterized in that** the lens (1, 11) further comprises at least one tab (3, 13) realized as extension of a corresponding plug (2, 12), each tab (3, 13) bearing a respective hole (5,15) and being designed as a protrusion of each plug (2, 12) to the direction of the optic plate (20,200).

2. The lens (1, 11) according to claim 1, wherein said plugs (2, 12) are T-shaped.

3. The lens (1, 11) according to claim 1 or 2, wherein said plugs (2, 12) are flexible.

4. The lens (1) according to one of the preceding claims, having two tabs (3).

5. The lens (11) according to one of the claims 1 to 3, having only one tab (13).

6. The lens according to one of the preceding claims, having a 5 to 8 mm optic diameter and an overall diameter between 11 and 15 mm.

7. The lens according to one of the preceding claims, wherein the haptics (10,100) have a 0-13° anterior angulation with respect to the optic plate (20, 200).

8. The lens according to one of the preceding claims, realized with a hydrophilic material.

9. The lens according to one of the preceding claims, realized with a hydrophobic material.

10. The lens according to one of the preceding claims, having at least a not transparent or coloured portion.

11. The lens according to claim 10, wherein said at least a not transparent or coloured portion is one or more of the plugs (2, 12).

12. The lens according to claim 10 or 11, wherein said at least a not transparent or coloured portion is one or more of the haptics (10, 100).

## Patentansprüche

1. Intraokularlinse (1, 11) für eine nahtlose sklerale Fixierung in einer Hinterkammer, umfassend eine Optikplatte (20, 200) und zwei Haptiken (10, 100), wobei jede Haptik (10, 100) an ihrem Endpunkt jeweils eine Steckvorrichtung (2, 12) für die Verankerung an der Sklera aufweist, wobei jede Steckvorrichtung (2, 12) ein Loch (4, 14) trägt;
**dadurch gekennzeichnet, dass** die Linse (1, 11) ferner mindestens eine Lasche (3, 13) umfasst, die als Verlängerung einer entsprechenden Steckvorrichtung (2, 12) ausgeführt ist, wobei jede Lasche (3, 13) jeweils ein Loch (5, 15) trägt und als eine Vorstülpung jeder Steckvorrichtung (2, 12) in Richtung der Optikplatte (20, 200) gestaltet ist.

2. Linse (1, 11) nach Anspruch 1, wobei die Steckvorrichtungen (2, 12) T-förmig sind.

3. Linse (1, 11) nach Anspruch 1 oder 2, wobei die Steckvorrichtungen (2, 12) flexibel sind.

4. Linse (1) nach einem der vorstehenden Ansprüche, die zwei Laschen (3) aufweist.

5. Linse (11) nach einem der Ansprüche 1 bis 3, die nur eine Lasche (13) aufweist.

6. Linse nach einem der vorstehenden Ansprüche, die einen optischen Durchmesser von 5 bis 8 mm und einen Gesamtdurchmesser zwischen 11 und 15 mm aufweist.

7. Linse nach einem der vorstehenden Ansprüche, wobei die Haptiken (10, 100) eine anteriore Winkelstellung von 0-13° in Bezug auf die Optikplatte (20, 200) aufweisen.

8. Linse nach einem der vorstehenden Ansprüche, die aus einem hydrophilen Material ausgeführt ist.

9. Linse nach einem der vorstehenden Ansprüche, die aus einem hydrophoben Material ausgeführt ist.

10. Linse nach einem der vorstehenden Ansprüche, die mindestens einen nicht transparenten oder gefärbten Anteil aufweist.

11. Linse nach Anspruch 10, wobei der mindestens eine nicht transparente oder gefärbte Anteil eine oder mehrere der Steckvorrichtungen (2, 12) ausmacht.

12. Linse nach Anspruch 10 oder 11, wobei der mindestens eine nicht transparente oder gefärbte Anteil eine oder mehrere der Haptiken (10, 100) ausmacht.

## Revendications

1. Lentille intraoculaire (1, 11) destinée à une fixation sclérale sans suture de chambre postérieure, comprenant une plaque optique (20, 200) et deux haptiques (10, 100), chaque haptique (10, 100) ayant, au niveau de son extrémité, un embout (2, 12) respectif destiné à l'ancrage à la sclère, chaque embout (2, 12) portant un trou (4, 14) ;
**caractérisée en ce que** la lentille (1, 11) comprend en outre au moins une patte (3, 13) réalisée en tant qu'extension d'un embout (2, 12) correspondant, chaque patte (3, 13) portant un trou (5, 15) respectif et étant conçue en tant que partie saillante de chaque embout (2, 12) dans la direction de la plaque optique (20, 200).

2. Lentille (1, 11) selon la revendication 1, dans laquelle lesdits embouts (2, 12) sont en forme de T.

3. Lentille (1, 11) selon la revendication 1 ou 2, dans laquelle lesdits embouts (2, 12) sont flexibles.

4. Lentille (1) selon l'une des revendications précédentes, ayant deux pattes (3).

5. Lentille (11) selon l'une des revendications 1 à 3, n'ayant qu'une seule patte (13).

6. Lentille selon l'une des revendications précédentes, ayant un diamètre optique de 5 à 8 mm et un diamètre global entre 11 et 15 mm.

7. Lentille selon l'une des revendications précédentes, dans laquelle les haptiques (10, 100) ont une angulation antérieure de 0 à 13° par rapport à la plaque optique (20, 200).

8. Lentille selon l'une des revendications précédentes, réalisée avec un matériau hydrophile.

9. Lentille selon l'une des revendications précédentes, réalisée avec un matériau hydrophobe.

10. Lentille selon l'une des revendications précédentes, ayant au moins une partie non transparente ou colorée.

11. Lentille selon la revendication 10, dans laquelle ladite au moins une partie non transparente ou colorée est un ou plusieurs des embouts (2, 12).

12. Lentille selon la revendication 10 ou 11, dans laquelle ladite au moins une partie non transparente ou colorée est une ou plusieurs des haptiques (10, 100).
